# FASCICULE DE BREVET EUROPEEN

(11) **EP 2 791 098 B1**
(45) Date de publication et mention de la délivrance du brevet: **19.04.2017**
(21) Numéro de dépôt: 12808776.4
(22) Date de dépôt: 13.12.2012
(51) Int. Cl.: C07C 45/80, C07C 47/58

(54) **PROCEDE DE PURIFICATION DE LA VANILLINE PAR EXTRACTION LIQUIDE-LIQUIDE**
VERFAHREN ZUR REINIGUNG VON VANILLIN DURCH FLÜSSIG-FLÜSSIG-EXTRAKTION
METHOD FOR PURIFYING VANILLIN BY LIQUID-LIQUID EXTRACTION

(30) Priorité: 15.12.2011 FR 1161681
(43) Date de publication de la demande: 22.10.2014
(73) Titulaire: Rhodia Operations, 75009 Paris (FR)
(72) Inventeur: VIBERT, Martine, 69006 Lyon (FR); COCHENNEC, Corine, 38500 Voiron (FR); ETCHEBARNE, Alain, 79500 Melle (FR)
(74) Mandataire: Menville, Laure
(86) Numéro de dépôt international: PCT/EP2012/075456
(87) Numéro de publication internationale: WO 2013/087795

(56) Documents cités:
- CN-A- 1 250 083
- US-A- 4 021 493

## Description

La présente invention a pour objet un procédé de purification de la vanilline et de ses dérivés, notamment de vanilline naturelle issue de fermentation. Par dérivés de vanilline, on entend au sens de la présente invention le glycoside de vanilline.

La vanilline est un produit largement utilisé dans de nombreux domaines d'application en tant qu'arôme et/ou parfum. Ainsi, la vanilline se trouve abondamment consommée dans l'industrie alimentaire et animale mais elle a aussi des applications dans d'autres domaines tels que par exemple, la pharmacie ou la parfumerie. Il s'ensuit que c'est un produit de grande consommation.

Dans le cadre de l'utilisation d'arômes, il est de plus en plus important que les produits utilisés soient désignés comme "produits naturels". Selon les règlementations en Europe et aux Etats-Unis, cela signifie que le composé doit être obtenu par des procédés physiques, enzymatiques ou microbiologiques et uniquement à partir de matières d'origine végétale ou animale.

Ainsi, les recherches concernant la purification de vanilline sont centrées autour de l'utilisation de matières brutes naturelles, peu coûteuses et renouvelables. Dans ce cadre, de nombreuses demandes de brevet concernent la production microbienne ou enzymatique de vanilline. En général, un précurseur approprié est transformé en vanilline par un microorganisme ou une enzyme. Parmi les précurseurs utilisés, on peut notamment citer l'eugénol ou molécules apparentées, (par exemple l'isoeugénol), l'acide férulique, la curcumine ou la résine de benjoin du Siam. Toutefois, les rendements obtenus pour ces procédés sont généralement très faibles.

Parmi tous ces procédés, on peut par exemple citer le procédé de fermentation décrit dans la demande EP 0 761 817, décrivant l'utilisation de deux souches du genre *Amycolatopsis* pour la fermentation à partir d'acide férulique.

La demande de brevet CN 1250083 décrit un procédé d'extraction de la vanilline comprenant une étape d'extraction de la vanilline d'une solution de benzène à l'aide d'une solution aqueuse alcaline.

Il existe toujours un besoin important d'optimiser les procédés de préparation de vanilline et de glycoside de vanilline, notamment de vanilline naturelle.

La présente invention a donc pour but de fournir un procédé de purification de vanilline avec un rendement très élevé en vanilline, notamment supérieur à 80%, voire supérieur à 90%.

La présente invention a également pour but de fournir un procédé de purification de vanilline permettant d'obtenir de la vanilline avec un titre très élevé, notamment supérieur à 97%, voire égal à 100%.

La présente invention a également pour but de fournir un procédé permettant d'obtenir de la vanilline ou de glycoside de vanilline à l'échelle industrielle sous forme de produit commercialisé, le produit ainsi obtenu étant naturel au sens de la réglementation.

Le procédé selon l'invention et les étapes qu'il met en oeuvre sont réalisés en conformité avec le règlement arôme n°1334/2008 /CE.

La présente invention concerne donc un procédé de purification de vanilline et de glycoside de vanilline, à partir d'une solution de vanilline ou de glycoside de vanilline dans un solvant S1 contenant des impuretés, comprenant les étapes suivantes :
a) une étape d'évaporation du solvant S1 en présence d'eau pour obtenir une solution aqueuse de vanilline ou de glycoside de vanilline,
b) une étape d'extraction liquide/liquide par mise en contact de la solution aqueuse obtenue à l'issue de l'étape a) avec un solvant S2, à un pH supérieur à 8 et inférieur à 10, pour obtenir une phase organique et une phase aqueuse contenant de la vanilline ou du glycoside de vanilline et du solvant S2 résiduel ;
c) une étape de précipitation, à un pH compris de 4 à 7,5, de la vanilline ou du glycoside de vanilline contenu(e) dans la phase aqueuse obtenue à l'issue de l'étape b), et
d) une étape d'isolement de la vanilline ou du glycoside de vanilline.

Le procédé de la présente invention consiste à purifier une solution de vanilline ou de glycoside de vanilline contenant des impuretés et un solvant (S1). Parmi ces impuretés, on peut citer par exemple l'acide benzoïque, l'alcool vanillique, le gaiacol, et leurs mélanges. La solution de vanilline peut encore éventuellement comprendre d'autres impuretés, en particulier, l'acide vanillique, l'acide férulique, des composés présentant un squelette avec plusieurs groupements phényles, en général deux ou trois groupements phényles, ainsi que d'autres composés lourds. Lesdits composés ayant deux groupements phényles sont appelés dimères dans la présente invention. Il s'agit en particulier du diphényleméthane et de ses dérivés présentant des substituants sur le(s) groupe(s) phényle(s). Les groupements phényles présents dans les dimères sont avantageusement séparés par une chaîne carbonée ou une chaîne présentant un hétéroatome, par exemple l'oxygène. Des dimères particuliers comprenant un motif férulique sont avantageusement présents dans ladite solution de vanilline. Des dimères avantageusement présents dans la solution de vanilline présentent les formules ci-dessous :

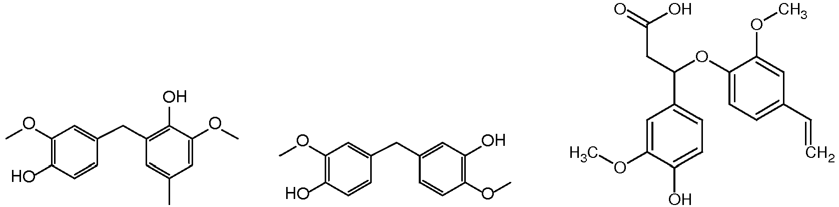

Le ratio pondéral impuretés/vanilline dans la solution de vanilline initiale (vanilline brute) est généralement compris entre 0,10 et 0,35, de préférence entre 0,15 et 0,35. Ainsi, le procédé de la présente invention consiste à éliminer ces impuretés afin d'augmenter le titre final en vanilline.

Cette solution de vanilline à purifier est également dénommée "solution de vanilline de départ" ou "solution de vanilline initiale" ou encore "vanilline brute".

Le procédé selon la présente invention permet d'obtenir de la vanilline sous forme solide avec un titre élevé, à partir d'une solution impure de vanilline.

Selon un mode de réalisation avantageux du procédé selon la présente invention, la solution de vanilline dans le solvant S1 est issue d'un procédé de fermentation. A titre de procédé de fermentation, on peut notamment citer le procédé décrit dans EP 0 761 817.

Selon un mode de réalisation, la vanilline brute est une solution de vanilline, contenant les impuretés formées lors de la fermentation de l'acide férulique et de la stabilisation de la fermentation.

Le procédé selon l'invention est également adapté à la purification d'un dérivé de vanilline : le glycoside de vanilline, notamment le glycoside de vanilline d'origine naturelle extrait des gousses de vanille.

Le procédé selon l'invention est mis en oeuvre selon un fonctionnement en mode continu ou selon un fonctionnement en mode discontinu.

Lorsqu'il est mis en oeuvre selon un fonctionnement discontinu, le procédé selon l'invention, en particulier lesdites étapes a), b) et c), est préférentiellement réalisé dans une même enceinte laquelle est avantageusement équipée d'au moins une colonne à distiller surmontée d'au moins un condenseur.

### Etape a) - évaporation du solvant S1 en présence d'eau

Le procédé de la présente invention comprend une étape a) consistant à éliminer par évaporation le solvant S1 présent dans la solution de vanilline initiale ou la solution du glycoside de vanilline. Conformément au procédé selon l'invention, ladite étape d'évaporation est réalisée en présence d'eau. De manière préférée, le solvant S1 présente une température d'ébullition inférieure à 100°C ou forme un azéotrope, avec l'eau, de température d'ébullition inférieure à 100°C.

Parmi les solvants S1, on peut par exemple citer les solvants organiques autorisés par la règlementation tels que les acétates d'alkyle (acétate d'éthyle, acétate de propyle, acétate d'isopropyle), la MEK (méthyl-éthylcétone), le cyclohexane, le dichlorométhane. Le solvant S1 peut encore être l'eau.

Le solvant S1 peut également être un mélange de solvants organiques, notamment un mélange de solvants organiques cités ci-dessus ou un mélange d'eau et d'un solvant organique.

Selon un mode de réalisation préféré, le solvant S1 est l'acétate d'éthyle.

De préférence, dans la solution de vanilline initiale, la concentration massique de la vanilline est comprise de 10% à 60%, plus préférentiellement de 10 à 40%, et encore plus préférentiellement de 10 à 35% par rapport à la masse totale de ladite solution. L'étape a) selon la présente invention consiste à éliminer le solvant S1 pour obtenir une solution aqueuse de vanilline dans laquelle la concentration massique de la vanilline est comprise de préférence entre 5% et 40%, de manière plus préférée entre 5% et 35% et de manière encore plus préférée entre 5% et 25% par rapport à la masse totale de ladite solution.

Conformément au procédé selon l'invention, l'étape d'évaporation a) est réalisée en présence d'eau laquelle est ajoutée à la solution de vanilline initiale avant et/ou pendant la mise en oeuvre de ladite étape d'évaporation.

Selon un mode de réalisation préféré, dans le cadre de l'étape a), le solvant S1 de la vanilline brute est éliminé par évaporation, par exemple par distillation ou au moyen d'un évaporateur, en présence d'eau, de sorte que la vanilline et les impuretés se retrouvent finalement en phase aqueuse sous forme soluble ou non. Dans le cas d'une évaporation par distillation, le solvant S1 peut être distillé à pression atmosphérique ou sous vide ou encore à pression atmosphérique puis sous vide.

L'eau peut être ajoutée en une ou plusieurs fois à la solution de vanilline initiale. Il est préférable d'utiliser de l'eau alimentaire (par exemple de l'eau de ville). On peut également utiliser une eau alimentaire recyclée, provenant du procédé selon la présente invention (par exemple les eaux de lavages, les eaux mères de cristallisation ou de précipitation) comme décrit plus loin.

La quantité d'eau avantageusement ajoutée à la solution de vanilline initiale avant et/ou pendant la mise en oeuvre de ladite étape d'évaporation a) est telle que la concentration massique en vanilline dans la solution aqueuse obtenue à l'issue de l'étape a) est comprise avantageusement entre 5% et 40% en poids, très avantageusement entre 5 et 35% en poids et encore plus avantageusement entre 5 et 25% en poids par rapport à la masse totale de ladite solution. De préférence, cette concentration massique est comprise de 10% à 15% en poids. La solution aqueuse de vanilline obtenue à l'issue de ladite étape a) contient les impuretés formées lors de la fermentation et plus généralement celles présentes dans la solution de vanilline soumise à l'étape d'évaporation.

De préférence, ladite étape d'évaporation a) est effectuée à une température comprise entre 60 et 120°C et très préférentiellement entre 80 et 120°C.

### Etape b) - extraction liquide/liquide

A l'issue de l'étape a), la solution aqueuse de vanilline ou de glycoside de vanilline obtenue est soumise à une étape d'extraction liquide/liquide dans des conditions de pH particulières.

Le solvant utilisé pour cette étape d'extraction est désigné ci-après solvant S2.

Cette étape est effectuée à pH contrôlé afin de séparer certaines impuretés de la vanilline par différence de pKa. En particulier, ladite étape b) est mise en oeuvre de manière à extraire les impuretés formées par des espèces ayant un pKa plus élevé que celui de la vanilline. Il s'agit par exemple de l'alcool vanillique, du gaiacol et de certains dimères ou de composés lourds. L'extraction selon ladite étape b) peut être totale ou partielle. Conformément à ladite étape b) du procédé selon l'invention, le pH est choisi de manière à obtenir un rendement élevé en vanilline. Ainsi, le pH est strictement supérieur à 8 et inférieur à 10.

Lors de cette étape d'extraction à pH contrôlé, les espèces protonées sont extraites par le solvant S2 et la couche organique comprend majoritairement le solvant S2. La vanilline reste alors en phase aqueuse sous forme de vanillinate. Pour la suite du procédé selon la présente invention, on utilise la phase aqueuse ainsi obtenue, c'est-à-dire que les étapes ultérieures à ladite étape d'extraction liquide-liquide sont effectuées à partir de la phase aqueuse contenant le vanillinate.

Selon un mode de réalisation, le solvant d'extraction S2 est différent du solvant S1 présent dans la solution de vanilline initiale.

Selon un autre mode de réalisation, les solvants S1 et S2 sont identiques. Ce mode est avantageux car il permet en particulier de ne procéder qu'à l'évaporation partielle dudit solvant selon ladite étape a) du procédé selon l'invention et d'utiliser la partie non évaporée pour la mise en oeuvre de ladite étape d'extraction liquide-liquide.

Conformément au procédé selon l'invention, le solvant S1 et le solvant S2 sont préférentiellement choisis parmi les solvants autorisés par la réglementation en vigueur selon la directive 2009/32/CE et la directive 2010/59/UE concernant les solvants d'extraction utilisés dans la fabrication des denrées alimentaires et de leurs ingrédients.

Conformément à ladite étape b) du procédé selon l'invention, le solvant d'extraction S2 présente une solubilité dans l'eau nulle ou très faible à moyenne. Plus précisément, la teneur pondérale maximale dudit solvant S2 dans l'eau est égale à 70 g/l. Il va de soi que le solvant S2 est un solvant organique.

De préférence, le solvant S2 est un solvant présentant une solubilité dans l'eau faible ou très faible, c'est-à-dire que sa teneur pondérale maximale dans l'eau est égale à 50 g/l, et préférentiellement sa teneur pondérale maximale dans l'eau est égale à 20 g/l. Le solvant S2 peut également être un solvant insoluble dans l'eau.

Ledit solvant d'extraction S2 présente avantageusement une température d'ébullition inférieure à 200°C, préférentiellement inférieure à 150°C.

Parmi les solvants S2 employés pour la mise en oeuvre de ladite étape b) du procédé selon l'invention, on peut par exemple mentionner le dichlorométhane, le cyclohexane, l'acétate d'éthyle, l'acétate de propyle, l'acétate d'isopropyle, l'acétate de n-butyle, l'acétate d'iso-amyle, la méthyl isobutyl cétone (MIBK), le butanol, le Rhodiasolv® RPDE (mélange de diméthyladipate, diméthylsuccinate et de diméthylglutarate) ou un mélange de ces solvants.

Selon un mode de réalisation avantageux, le solvant S2 est choisi parmi les solvants alimentaires. De préférence, le solvant S2 est choisi dans le groupe constitué de l'acétate d'éthyle, de l'acétate de propyle, de l'acétate d'isopropyle, de l'acétate de n-butyle, de l'acétate d'iso-amyle, de la méthyl isobutyl cétone (MIBK), et de leurs mélanges. De manière encore plus préférée, le solvant S2 est l'acétate d'isopropyle.

Afin d'obtenir les conditions de pH particulières décrites ci-dessus, selon un mode de réalisation, l'étape b) du procédé selon l'invention comprend l'addition d'une base, ladite base pouvant être soit une base faible soit une base forte. D'une manière avantageuse, ladite base est choisie parmi les bases minérales, et plus particulièrement les bases minérales solubles dans l'eau. En particulier, ladite base est choisie dans le groupe constitué des hydroxydes de métaux alcalins, des hydroxydes de métaux alcalino-terreux, des bicarbonates de métaux alcalins, des bicarbonates de métaux alcalino-terreux, des hydrogénocarbonates de métaux alcalins, des hydrogénocarbonates de métaux alcalino-terreux, des phosphates de métaux alcalins, des phosphates de métaux alcalino-terreux, des hydrogénophosphates de métaux alcalins, des hydrogénophosphates de métaux alcalino-terreux, et de leurs mélanges. De manière très avantageuse, ladite base est choisie parmi les bases minérales suivantes : NaOH, KOH et Na₂CO₃. Préférentiellement, on utilise NaOH ou KOH à titre de base, et plus particulièrement NaOH.

Cette étape d'addition de la base permet d'ajuster le pH pour l'étape d'extraction liquide-liquide à une valeur de pH supérieur à 8 et inférieur à 10. De manière préférée, ladite étape d'extraction selon l'étape b) du procédé selon l'invention est réalisée à un pH compris entre 8,1 et 9,5, très préférentiellement entre 8,3 et 9,5 et encore plus préférentiellement entre 8,5 et 9. Le contrôle du pH pour la mise en oeuvre de ladite étape b) conduit, à l'issue de la mise en oeuvre du procédé de l'invention, à la production d'une vanilline de haute pureté avec un rendement optimal.

Conformément à l'étape b) du procédé selon l'invention, l'addition de ladite base à la solution de vanilline est effectuée avant l'addition du solvant S2 ou après l'addition du solvant S2. Selon un mode de réalisation préféré, la base est ajoutée rapidement dans la solution de vanilline, de préférence en une seule fois.

Selon un mode de réalisation du procédé de la présente invention, la base est ajoutée dans la solution de vanilline, contenant ou non le solvant S2, à une température comprise de 15 °C à 60 °C, et de préférence comprise entre 30 et 50 °C.

Selon un mode de réalisation du procédé de la présente invention, la base avantageusement utilisée pour la mise en oeuvre de ladite étape b) est diluée dans l'eau selon une concentration comprise de 5% à 30% en poids par rapport au poids de ladite solution aqueuse dans laquelle est diluée ladite base.

Conformément au procédé selon l'invention, ladite étape b) est réalisée selon un rapport massique entre la masse de solvant S2 et la masse de vanilline avantageusement compris de 0,2 à 3, de préférence de 0,5 à 3, et préférentiellement de 0,6 à 1,2.

L'étape d'extraction liquide-liquide est de préférence effectuée à pression atmosphérique et à une température comprise entre 15 et 40 °C, préférentiellement entre 20 et 30 °C. De manière préférée, ladite étape d'extraction est précédée d'une étape de refroidissement.

Selon un mode de réalisation particulier, on ajoute la base dans la solution de vanilline obtenue à l'issue de ladite étape a) à une température comprise de 15 à 60 °C, de préférence comprise de 30 °C à 50 °C, puis on abaisse la température jusqu'à une température préférentiellement comprise entre 15 et 40 °C, très préférentiellement comprise entre 20 °C et 30 °C, avant d'ajouter le solvant S2 pour l'extraction.

A l'issue de l'étape b), on obtient une phase organique et une phase aqueuse.

La phase organique contient une partie des impuretés, notamment le gaiacol, l'alcool vanillique et des dimères, et le solvant S2 tandis que la phase aqueuse contient la vanilline dans l'eau, l'autre partie des impuretés, par exemple l'acide férulique, l'acide vanillique, l'acide benzoïque et/ou les dimères particuliers comprenant un motif férulique, ainsi que du solvant S2 résiduel. Le solvant S2 présent dans la phase organique, préférentiellement libérée des impuretés, est avantageusement recyclé en amont de ladite étape b).

Selon un fonctionnement en mode discontinu du procédé selon l'invention, on laisse ensuite préférentiellement décanter la phase organique pour récupérer la phase aqueuse.

Selon un fonctionnement en mode continu du procédé selon l'invention, ladite étape d'extraction liquide/liquide est avantageusement mise en oeuvre par l'utilisation d'une série de mélangeurs décanteurs ou d'au moins une colonne d'extraction liquide/liquide agitée, pulsée ou garnie. Elle peut encore être mise en oeuvre en mode continu par l'emploi d'un mélangeur statique puis d'un séparateur centrifuge pour réaliser la séparation des phases organique et aqueuse en mode continu.

Selon un mode de réalisation avantageux du procédé selon la présente invention, on soumet la phase aqueuse obtenue à l'issue de l'étape d'extraction liquide-liquide à une étape réalisant l'élimination du solvant S2 résiduel (étape dite de « stripping ») de manière à améliorer la qualité de la vanilline obtenue à l'issue du procédé de l'invention.

Une telle étape de stripping est effectuée dans des conditions douces, notamment par injection d'un fluide gazeux (par exemple de la vapeur d'eau ou du diazote, de préférence du diazote) et/ou mise sous vide de l'enceinte où est opéré le procédé selon l'invention.

Préférentiellement, l'étape de stripping est effectuée sous vide. Elle est avantageusement mise en oeuvre à une température comprise entre 20 °C et 50 °C. La durée de ladite étape est par exemple de 40 à 120 minutes.

### Etape c) - précipitation

A l'issue de l'étape b), la vanilline ou le glycoside de vanilline est présent(e) en solution aqueuse sous forme vanillinate.

Cette solution contient une grande partie des impuretés présentes dans la vanilline brute, en particulier les espèces dont le pKa est inférieur à celui de la vanilline. Il s'agit en particulier de l'acide férulique, de l'acide vanillique, de l'acide benzoïque et des dimères particuliers comprenant un motif férulique.

L'étape c) selon le procédé de l'invention est effectuée à un pH contrôlé pour abaisser le pH de la solution contenant la vanilline sous forme vanillinate. En abaissant le pH, on précipite la vanilline, les impuretés restant dans la solution, dite jus mères.

L'étape c) du procédé selon l'invention est effectuée à un pH compris entre 4 et 7,5. Ces conditions de précipitation de la vanilline permettent d'obtenir un rendement approprié en vanilline, et également un titre en vanilline minimal de 97% dans le précipité obtenu à l'issue de l'étape de précipitation. Le pH lors de l'étape de précipitation est préférentiellement compris entre 5 et 7, très préférentiellement entre 5,7 et 6,5 et encore plus préférentiellement entre 5,8 et 6,3.

Ladite étape de précipitation selon le procédé de l'invention est effectuée au moyen d'un acide aqueux faible ou fort, lequel est introduit dans ladite phase aqueuse obtenue à l'issue de ladite étape b) du procédé selon l'invention. De préférence, on utilise un acide ne réagissant pas avec la vanilline. Parmi les acides, on peut notamment citer les acides dont les sels formés sont solubles dans l'eau. Selon un mode de réalisation préféré, l'étape de précipitation susmentionnée est effectuée en présence d'acide sulfurique.

Selon un mode de réalisation du procédé selon l'invention, ladite étape c) est de préférence effectuée à une température comprise de 15 °C à 40 °C, et préférentiellement de 25 °C à 40 °C. De manière avantageuse, ladite étape de précipitation est effectuée sous pression atmosphérique. Conformément audit mode de réalisation du procédé selon l'invention, ladite étape c) comprend l'addition d'un acide, présent en solution aqueuse, dans ladite phase aqueuse obtenue par décantation à l'issue de ladite étape b) puis, de manière préférentielle, le refroidissement du milieu aqueux dans lequel la vanille précipite. Le refroidissement est préférentiellement réalisé jusqu'à atteindre une température avantageusement inférieure ou égale à 20 °C, de préférence inférieure ou égale à 15 °C et de manière préférée une température comprise entre 5 et 15 °C.

Selon un autre mode de réalisation du procédé selon l'invention, l'addition d'acide à ladite phase aqueuse de vanilline issue de ladite étape b) est effectuée préférentiellement à une température comprise entre 50 et 95 °C, très préférentiellement entre 50 et 70 °C et à une pression préférentiellement comprise entre 0,012 et 0,085 MPa, très préférentiellement entre 0,012 et 0,03 MPa, et est suivie d'un refroidissement contrôlé jusqu'à une température comprise entre 0 et 5 °C. Ledit refroidissement est avantageusement accompagné d'une diminution contrôlée de la pression jusqu'à une pression comprise entre 0,006 MPa et 0,008 MPa. Le refroidissement est avantageusement opéré au moyen d'un échangeur interne et/ou par circulation d'un fluide caloporteur (en particulier l'eau) au sein d'une double enveloppe dont le réacteur où est réalisé ladite étape c) est équipé.

### Etape d) - isolement de la vanilline

La vanilline ou le glycoside de vanilline obtenu(e) sous forme de précipité à l'issue de ladite étape c) est isolée selon l'étape d) du procédé selon l'invention en vue d'améliorer sa purification. Ladite étape d) consiste avantageusement au moins en une étape de récupération de la vanilline solide sur filtre ou essoreuse, suivie par une ou plusieurs étapes de lavage à l'eau, et préférentiellement suivie d'au moins une étape de séchage.

La vanilline solide issue de ladite étape c) de précipitation est récupérée sur filtre ou sur essoreuse. Afin d'éliminer les impuretés résiduelles, notamment les sels minéraux dont des sulfates, un ou plusieurs lavages à l'eau peuvent être nécessaires.

La vanilline est ensuite avantageusement séchée pour être vendue telle qu'elle. Elle peut encore être broyée et/ou recristallisée dans l'eau ou dans un mélange eau/alcool selon un processus connu.

Les étapes de lavage et de séchage mises en oeuvre dans le cadre de la présente invention sont effectuées selon des protocoles classiques bien connus de l'homme du métier.

Aussi, selon un mode de réalisation, le procédé de la présente invention peut comprendre, à l'issue de l'étape d), une étape de recristallisation de la vanilline dans l'eau ou dans un mélange alcool/eau. La vanilline ainsi obtenue est sous forme de cristaux blancs.

Dans le cadre de la présente invention, il est possible d'encore améliorer les rendements en effectuant une ou plusieurs étape(s) supplémentaire(s) de recyclage.

Ces étapes supplémentaires consistent à recycler divers effluents obtenus au cours du procédé de l'invention, par exemple au cours de l'étape d'extraction, de précipitation ou de lavage.

Par exemple, il est possible de recycler les eaux de lavage (c'est-à-dire l'eau récupérée après les étapes de lavage) et de les utiliser comme eau du procédé, c'est-à-dire avec l'eau ajoutée avant et/ou la mise en oeuvre de ladite étape d'évaporation a) selon le procédé de l'invention.

Il est également possible de récupérer une partie de la vanilline contenue dans les jus mères obtenus à l'issue de l'étape de précipitation. Cette récupération peut être effectuée soit par modification du pH puis extraction avec un solvant, soit par reconcentration et précipitation, soit par reconcentration et extraction.

Il est aussi possible de récupérer, par lavage, une partie de la vanilline contenue dans la phase organique à l'issue de l'étape d'extraction.

Les exemples ci-après illustrent davantage la présente invention mais ne sont en aucun cas limitatifs.

### Exemple 1 : procédé de purification de vanilline dans lequel l'étape d'extraction est réalisée au moyen de dichlorométhane (DCM)

Dans une enceinte munie d'une colonne à distiller et d'un condenseur, on introduit 1 594 g de solution de vanilline brute contenant 19,3% en poids de vanilline, 1,4% en poids d'alcool vanillique, 6 g (0,4 % poids) d'acide benzoïque et 18,5 g (1,1 % poids) d'autres impuretés, notamment le gaiacol, les dimères de type diphényleméthane et les dimères à motif férulique ainsi que des composés lourds. Le solvant présent dans cette solution est l'acétate d'éthyle et représente le reste pondéral de la solution de vanilline brute.

On a ajouté dans cette solution 860 g d'eau puis le solvant constitué d'acétate d'éthyle a été distillé à pression atmosphérique puis sous vide à une température égale à 100°C et récupéré en tête (1404g). On a enfin ajouté 1 800 g d'eau dans la solution contenant la vanilline après évaporation. Une quantité égale à 360 g de soude (22% poids dans l'eau) est ajoutée à cette solution de vanilline et on a alors obtenu une solution à un pH égal à 8,9. Cette solution a été maintenue à une température égale à 34 °C puis est laissée refroidir à 24 °C.

Ensuite, on a ajouté 200 g de dichlorométhane (DCM) puis effectué l'étape d'extraction à 24 °C.

La phase organique ainsi obtenue contient majoritairement le dichlorométhane mais également 6 g de vanilline et 9 g d'impuretés, en particulier l'alcool vanillique, le gaiacol, les dimères de type diphényleméthane et autres lourds.

La phase aqueuse contenant la vanilline sous forme de vanillinate et du dichlorométhane résiduel a ensuite été strippée sous vide (150 mm Hg = 0,2 bar) à 35 °C sous injection d'azote durant 2h.

On a ensuite ajouté dans la phase aqueuse de l'acide sulfurique (H₂SO₄ 50% poids dans l'eau) de manière à obtenir une solution ayant un pH = 6,4 à 29°C. On a alors obtenu, en abaissant la température jusqu'à 20°C, un précipité qui a été filtré pour récupérer 2 750 g de jus mères contenant 30 g de vanilline et des impuretés, en particulier l'acide benzoïque et des dimères particuliers comprenant un motif férulique.

Enfin, le solide obtenu a été lavé par 750 g d'eau en 2 fois.

La vanilline ainsi obtenue avait un titre de 98,5% et contenait moins de 0,1% en poids de Na₂SO₄.

Le rendement global (rapport pondéral {vanilline purifiée / vanilline présente dans la solution de vanilline brute}) en vanilline sans recyclage de flux était de 85%.

Avec recyclage des eaux de lavages et d'une partie de la vanilline des eaux mères, le rendement était de 94% avec un titre final de 98%.

### Exemple 2 : procédé de purification de vanilline dans lequel l'étape d'extraction est réalisée au moyen d'acétate d'isopropyle

Dans une enceinte munie d'une colonne à distiller et d'un condenseur, on a utilisé 200 g de solution de vanilline brute contenant 19,4% en poids de vanilline, 1,4% en poids d'alcool vanillique, 1% poids d'acide benzoïque et 5 g d'autres impuretés, en particulier l'acide vanillique, le gaiacol, des dimères de type diphényleméthanes et des dimères particulier comprenant un motif férulique ainsi que des composés lourds. Le solvant présent dans cette solution est l'acétate d'éthyle et représente le reste pondéral de la solution de vanilline brute.

On a ajouté dans cette solution 290 g d'eau puis le solvant constitué d'acétate d'éthyle a été distillé à pression atmosphérique à une température égale à 100°C puis est récupéré en tête (180g). On a ajouté à cette solution aqueuse de vanilline 40 g de soude (22% poids dans l'eau) et on a alors obtenu une solution à un pH égal à 8,6. Cette solution a été maintenue à une température égale à 40 °C puis est laissée refroidir à 30 °C.

Ensuite, on a ajouté 39 g d'acétate d'isopropyle puis effectué l'étape d'extraction à 30 °C.

La phase organique ainsi obtenue contenait majoritairement l'acétate d'isopropyle mais également 1,4 g de vanilline et 2 g d'impuretés, en particulier l'alcool vanillique, le gaiacol, des dimères et des composés lourds.

La phase aqueuse contenant la vanilline sous forme de vanillinate et de l'acétate d'isopropyle résiduel a ensuite été strippée sous vide (150 mm Hg = 0,2 bar) à 35 °C sous injection d'azote durant 2h. Elle est ensuite refroidie jusqu'à environ 25 °C.

On a ensuite ajouté dans la phase aqueuse de l'acide sulfurique (H₂SO₄ 50% poids dans l'eau) de manière à obtenir une solution ayant un pH égal à 6 à 25 °C. On a alors obtenu, en abaissant la température jusqu'à 18 °C, un précipité qui a été filtré pour récupérer 290 g de jus mères contenant 2,5 g de vanilline et 6,4 g d'impuretés, en particulier l'acide vanillique, l'acide benzoïque et les dimères particuliers comprenant un motif férulique.

Enfin, le solide obtenu a été lavé par 200 g d'eau en 2 fois.

La vanilline ainsi obtenue avait un titre de 99% et contenait moins de 0,1% de Na₂SO₄.

Le rendement global en vanilline sans recyclage de flux était de 80%.

Avec recyclage des lavages et d'une partie de la vanilline des eaux mères, le rendement était de 83% avec un titre final de 99%.

### Exemple 3 (comparatif): procédé de purification de vanilline dans lequel l'étape d'extraction est réalisée au moyen d'acétate d'isopropyle à un pH = 10,5

Dans une enceinte munie d'une colonne à distiller et d'un condenseur, on a utilisé 200 g de solution de vanilline brute contenant 19,4% en poids de vanilline, 1,4% en poids d'alcool vanillique, 1% poids d'acide benzoïque et 5 g d'autres impuretés, en particulier l'acide vanillique, le gaiacol, des dimères de type diphényleméthanes et des dimères particuliers comprenant un motif férulique ainsi que des composés lourds. Le solvant présent dans cette solution est l'acétate d'éthyle et représente le reste pondéral de la solution de vanilline brute.

On a ajouté dans cette solution 290 g d'eau puis le solvant constitué d'acétate d'éthyle a été distillé à pression atmosphérique à une température égale à 100°C puis est récupéré en tête (182g). On a ajouté à cette solution aqueuse de vanilline de la soude à 22% jusqu'à obtenir une solution à un pH égal à 10,5. Cette solution de couleur soutenue brun orangé a été maintenue à une température égale à 40 °C puis est laissée refroidir à 30 °C.

Ensuite, on a ajouté 39 g d'acétate d'isopropyle puis effectué l'étape d'extraction à 30 °C.

La phase organique ainsi obtenue contenait majoritairement l'acétate d'isopropyle et quelques impuretés en particulier de l'alcool vanillique, du gaiacol, des dimères et autres lourds.

La phase aqueuse contenant la vanilline sous forme de vanillinate et de l'acétate d'isopropyle résiduel a ensuite été strippée sous vide (150 mm Hg = 0,2 bar) à 35 °C sous injection d'azote durant 2h. Elle est ensuite refroidie jusqu'à environ 25 °C.

On a ensuite ajouté dans la phase aqueuse de l'acide sulfurique (H₂SO₄ 50% poids dans l'eau) de manière à obtenir une solution ayant un pH égal à 6 à 25 °C. On n'obtient pas de précipité même en abaissant la température jusqu'à 8 °C. L'analyse de la solution riche en vanilline (12.5% poids) montre que les impuretés organiques sont nombreuses, en particulier la présence de gaiacol, d'alcool vanillique, de dimères et autres lourds est détectée. L'acide vanillique et l'acide benzoïque sont également présents parmi les impuretés dans la solution de vanilline obtenue.

L'étape d'acidification ne permet donc pas d'isoler la vanilline purifiée.

### Exemple 4 (comparatif) : procédé de purification de vanilline dans lequel l'étape d'extraction est réalisée au moyen d'acétate d'isopropyle) à un pH = 7,5

Dans une enceinte munie d'une colonne à distiller et d'un condenseur, on a utilisé 200 g de solution de vanilline brute contenant 19,4 % en poids de vanilline, 1,4% en poids d'alcool vanillique, 1% poids d'acide benzoïque et 5g d'autres impuretés en particulier l'acide vanillique, le gaiacol, des dimères de type diphényleméthanes et des dimères particuliers comprenant un motif férulique ainsi que des composés lourds. Le solvant présent dans cette solution est l'acétate d'éthyle et représente le reste pondéral de la solution de vanilline brute.

On a ajouté dans cette solution 290 g d'eau puis le solvant constitué d'acétate d'éthyle a été distillé à pression atmosphérique à une température égale à 100 °C puis est récupéré en tête. On a ajouté à cette solution aqueuse de vanilline de la soude à 22% jusqu'à obtenir une solution à un pH égal à 7,5. Cette solution a été maintenue à une température égale à 40 °C puis est laissée refroidir à 30 °C.

Ensuite, on a ajouté 39 g d'acétate d'isopropyle puis effectué l'étape d'extraction à 30 °C.

La phase organique ainsi obtenue (55g) contenait majoritairement l'acétate d'isopropyle et 31 % poids de vanilline, et des impuretés en particulier de l'alcool vanillique, du gaiacol, des dimères et autres lourds.

La phase aqueuse contenant la vanilline sous forme de vanillinate et de l'acétate d'isopropyle résiduel a ensuite été strippée sous vide (150 mm Hg = 0,2 bar) à 35 °C sous injection d'azote durant 2h. Elle est ensuite refroidie jusqu'à environ 25 °C.

On a ensuite ajouté dans la phase aqueuse de l'acide sulfurique (H₂SO₄ 50% poids dans l'eau) de manière à obtenir une solution ayant un pH égal à 6 à 25 °C.

On a alors obtenu, en abaissant la température jusqu'à 18 °C, un précipité qui a été filtré pour récupérer 242 g de jus mères contenant de l'acide benzoique, de l'acide vanillique et d'autres impuretés, notamment les dimères particuliers comprenant un motif férulique.

Enfin, le solide obtenu a été lavé par 200 g d'eau en 2 fois.

La vanilline ainsi obtenue (14g) avait un titre de 98,7%.

Le rendement global (rapport pondéral {vanilline purifiée / vanilline présente dans la solution de vanilline brute}) en vanilline, sans recyclage de flux, était de 35% seulement.

## Revendications

1. Procédé de purification de vanilline et de glycoside de vanilline, à partir d'une solution de vanilline ou de glycoside de vanilline dans un solvant S1 contenant des impuretés, comprenant les étapes suivantes :
a) une étape d'évaporation du solvant S1 en présence d'eau pour obtenir une solution aqueuse de vanilline ou de glycoside de vanilline ;
b) une étape d'extraction liquide/liquide par mise en contact de la solution aqueuse obtenue à l'issue de l'étape a) avec un solvant S2, à un pH supérieur à 8 et inférieur à 10, pour obtenir une phase organique et une phase aqueuse contenant de la vanilline ou du glycoside de vanilline et du solvant S2 résiduel ;
c) une étape de précipitation, à un pH compris de 4 à 7,5, de la vanilline ou du glycoside de vanilline contenu(e) dans la phase aqueuse obtenue à l'issue de l'étape b), et
d) une étape d'isolement de la vanilline ou du glycoside de vanilline.

2. Procédé selon la revendication 1, tel que le solvant S2 est différent du solvant S1, ou les solvants S1 et S2 sont identiques.

3. Procédé selon la revendication 1 ou la revendication 2 tel que ledit solvant S1 présente une température d'ébullition inférieure à 100°C ou forme un azéotrope, avec l'eau, de température d'ébullition inférieure à 100°C.

4. Procédé selon l'une des revendications 1 à 3 tel que ledit solvant S1 est choisi parmi les acétates d'alkyle (acétate d'éthyle, acétate de propyle, acétate d'isopropyle), la MEK (méthyl-éthylcétone), le cyclohexane, le dichlorométhane.

5. Procédé selon l'une des revendications 1 à 4 tel que la teneur pondérale maximale dudit solvant S2 dans l'eau est égale à 70 g/l.

6. Procédé selon l'une des revendications 1 à 5 tel que ledit solvant S2 est choisi parmi le dichlorométhane, le cyclohexane, l'acétate d'éthyle, l'acétate de propyle, l'acétate d'isopropyle, l'acétate de n-butyle, l'acétate d'iso-amyle, la méthyl isobutyl cétone (MIBK), le butanol, le Rhodiasolv® RPDE (mélange de diméthyladipate, diméthylsuccinate et de diméthylglutarate) ou un mélange de ces solvants.

7. Procédé selon l'une des revendications 1 à 6 tel que ledit solvant S2 est l'acétate d'isopropyle.

8. Procédé selon l'une des revendications 1 à 7 tel que ladite étape b) comprend l'addition d'une base faible ou d'une base forte.

9. Procédé selon l'une des revendications 1 à 8 tel que ladite étape d'extraction liquide-liquide est réalisée à un pH compris entre 8,5 et 9.

10. Procédé selon l'une des revendications 1 à 9 tel que ladite base est diluée dans l'eau selon une concentration comprise de 5% à 30% en poids par rapport au poids de ladite solution aqueuse dans laquelle est diluée ladite base.

11. Procédé selon l'une des revendications 1 à 10 tel que ladite étape b) est réalisée selon un rapport massique entre la masse de solvant S2 et la masse de vanilline compris de 0,2 à 3

12. Procédé selon l'une des revendications 1 à 11 tel que ladite phase aqueuse obtenue à l'issue de l'étape d'extraction liquide-liquide est soumise à une étape réalisant l'élimination du solvant S2 résiduel.

13. Procédé selon l'une des revendications 1 à 12 tel que le pH lors de ladite étape de précipitation est compris entre 5,7 et 6,5.

14. Procédé selon l'une des revendications 1 à 13 tel que ladite étape d) consiste au moins en une étape de récupération de la vanilline solide sur filtre ou essoreuse, suivie par une ou plusieurs étapes de lavage à l'eau.

15. Procédé selon l'une des revendications 1 à 14 tel qu'il comprend à l'issue de l'étape d), une étape de recristallisation de la vanilline dans l'eau ou dans un mélange alcool/eau.

## Patentansprüche

1. Verfahren zur Reinigung von Vanillin und Vanillinglykosid aus einer Lösung von Vanillin oder Vanillinglykosid in einem Lösungsmittel S1, die Verunreinigungen enthält, bei dem man in den folgenden Schritten:
a) das Lösungsmittel S1 in Gegenwart von Wasser verdampft, um eine wässrige Lösung von Vanillin oder Vanillinglykosid zu erhalten;
b) eine Flüssig/Flüssig-Extraktion durchführt, indem man die nach Schritt a) erhaltene wässrige Lösung mit einem Lösungsmittel S2 bei einem pH von über 8 und unter 10 in Kontakt bringt, um eine organische Phase und eine wässrige Phase zu erhalten, die das Vanillin oder Vanillinglykosid und restliches Lösungsmittel S2 enthält;
c) das Vanillin oder Vanillinglykosid, das in der nach Schritt b) erhaltenen wässrigen Phase enthalten ist, bei einem pH von 4 bis 7,5 ausfällt und
d) das Vanillin oder Vanillinglykosid isoliert.

2. Verfahren nach Anspruch 1, wobei das Lösungsmittel S2 von dem Lösungsmittel S1 verschieden ist oder die Lösungsmittel S1 und S2 identisch sind.

3. Verfahren nach Anspruch 1 oder Anspruch 2, wobei das Lösungsmittel S1 einen Siedepunkt unter 100°C besitzt oder mit Wasser ein Azeotrop mit einem Siedepunkt unter 100°C bildet.

4. Verfahren nach einem der Ansprüche 1 bis 3, wobei das Lösungsmittel S1 aus Alkylacetaten (Ethylacetat, Propylacetat, Isopropylacetat), MEK (Methylethylketon), Cyclohexan, Dichlormethan ausgewählt ist.

5. Verfahren nach einem der Ansprüche 1 bis 4, wobei der maximale Gehalt, bezogen auf Gewicht, des Lösungsmittels S2 im Wasser gleich 70 g/l ist.

6. Verfahren nach einem der Ansprüche 1 bis 5, wobei das Lösungsmittel S2 aus Dichlormethan, Cyclohexan, Ethylacetat, Propylacetat, Isopropylacetat, n-Butylacetat, Isoamylacetat, Methylisobutylketon (MIBK), Butanol, Rhodiasolv® RPDE (Gemisch von Dimethyladipat, Dimethylsuccinat und Dimethylglutarat) oder einem Gemisch dieser Lösungsmittel ausgewählt wird.

7. Verfahren nach einem der Ansprüche 1 bis 6, wobei das Lösungsmittel S2 Isopropylacetat ist.

8. Verfahren nach einem der Ansprüche 1 bis 7, wobei Schritt b) die Zugabe einer schwachen Base oder einer starken Base umfasst.

9. Verfahren nach einem der Ansprüche 1 bis 8, wobei der Flüssig-Flüssig-Extraktionsschritt bei einem pH zwischen 8,5 und 9 durchgeführt wird.

10. Verfahren nach einem der Ansprüche 1 bis 9, wobei die Base in Wasser in einer Konzentration von 5 Gew.-% bis 30 Gew.-%, bezogen auf das Gewicht der wässrigen Lösung, in der die Base verdünnt wird, verdünnt wird.

11. Verfahren nach einem der Ansprüche 1 bis 10, wobei Schritt b) bei einem Massenverhältnis zwischen der Masse des Lösungsmittels S2 und der Masse an Vanillin von 0,2 bis 3 durchgeführt wird.

12. Verfahren nach einem der Ansprüche von 1 bis 11, wobei die nach dem Flüssig-Flüssig-Extraktionsschritt erhaltene wässrige Phase einem Schritt unterzogen wird, bei dem das restliche Lösungsmittel S2 entfernt wird.

13. Verfahren nach einem der Ansprüche 1 bis 12, wobei der pH-Wert während des Fällungsschritts zwischen 5,7 und 6,5 beträgt.

14. Verfahren nach einem der Ansprüche 1 bis 13, wobei Schritt d) aus mindestens einem Schritt der Gewinnung des festen Vanillins auf einem Filter oder einer Zentrifuge, gefolgt von einem oder mehreren Schritten des Waschens mit Wasser besteht.

15. Verfahren nach einem der Ansprüche 1 bis 14, das nach Schritt d) einen Schritt der Umkristallisation des Vanillins in Wasser oder in einem Alkohol/WasserGemisch umfasst.

## Claims

1. Process for purifying vanillin and vanillin glycoside, starting with a solution of vanillin or of vanillin glycoside in a solvent S1 containing impurities, comprising the following steps:
a) a step of evaporation of solvent S1 in the presence of water to obtain an aqueous solution of vanillin or of vanillin glycoside;
b) a step of liquid/liquid extraction by placing the aqueous solution obtained after step a) in contact with a solvent S2, at a pH above 8 and below 10, to obtain an organic phase and an aqueous phase containing vanillin or vanillin glycoside and residual solvent S2;
c) a step of precipitation, at a pH of between 4 and 7.5, of the vanillin or of the vanillin glycoside contained in the aqueous phase obtained after step b), and
d) a step of isolation of the vanillin or of the vanillin glycoside.

2. Process according to Claim 1, such that solvent S2 is different from solvent S1, or solvents S1 and S2 are identical.

3. Process according to Claim 1 or Claim 2, such that said solvent S1 has a boiling point of less than 100°C or forms an azeotrope, with water, having a boiling point of less than 100°C.

4. Process according to one of Claims 1 to 3, such that said solvent S1 is chosen from alkyl acetates (ethyl acetate, propyl acetate, isopropyl acetate), MEK (methyl ethyl ketone), cyclohexane and dichloromethane.

5. Process according to one of Claims 1 to 4, such that the maximum weight content of said solvent S2 in water is equal to 70 g/l.

6. Process according to one of Claims 1 to 5, such that said solvent S2 is chosen from dichloromethane, cyclohexane, ethyl acetate, propyl acetate, isopropyl acetate, n-butyl acetate, isoamyl acetate, methyl isobutyl ketone (MIBK), butanol, Rhodiasolv® RPDE (mixture of dimethyl adipate, dimethyl succinate and dimethyl glutarate), or a mixture of these solvents.

7. Process according to one of Claims 1 to 6, such that said solvent S2 is isopropyl acetate.

8. Process according to one of Claims 1 to 7, such that said step b) comprises the addition of a weak base or of a strong base.

9. Process according to one of Claims 1 to 8, such that said liquid-liquid extraction step is performed at a pH of between 8.5 and 9.

10. Process according to one of Claims 1 to 9, such that said base is diluted in water to a concentration from 5% to 30% by weight relative to the weight of said aqueous solution in which said base is diluted.

11. Process according to one of Claims 1 to 10, such that said step b) is performed with a mass ratio between the mass of solvent S2 and the mass of vanillin from 0.2 to 3.

12. Process according to one of Claims 1 to 11, such that said aqueous phase obtained after the liquid-liquid extraction step is subjected to a step for removing residual solvent S2.

13. Process according to one of Claims 1 to 12, such that the pH during said precipitation step is between 5.7 and 6.5.

14. Process according to one of Claims 1 to 13, such that said step d) consists of at least one step of recovering solid vanillin on a filter or spin-dryer, followed by one or more steps of washing with water.

15. Process according to one of Claims 1 to 14, such that it comprises, after step d), a step of recrystallization of the vanillin from water or from an alcohol/water mixture.
